# EUROPEAN PATENT APPLICATION

(11) **EP 4 717 693 A1**
(43) Date of publication of application: **01.04.2026**
(21) Application number: 25205157.8
(22) Date of filing: 28.09.2025
(51) Int. Cl.: C07D 307/80, A01N 43/12

(54) **SALTS OF 16,17-DIHYDROGIBBERELLIN-A5-13-ACETATE AND 2,3-DEHYDRO-16,17-DIHYDROGIBBERELLIN-A9, PREPARATIONS CONTAINING THESE SALTS AND USE THEREOF**

(30) Priority: 30.09.2024 CZ 20240374
(71) Applicant: Univerzita Palackého v Olomouci, 779 00 Olomouc (CZ)
(72) Inventor: Pospisil, Jiri, Olomouc (CZ); Fuksova, Marketa, Jihlava (CZ); Koprna, Radoslav, Olomouc (CZ); Hedden, Peter, Harpenden (GB); Jaworek, Pavel, Ostrava (CZ); Strnad, Miroslav, Olomouc (CZ)
(74) Representative: Harber IP s.r.o.

(57) **Abstract**

The invention relates to salts of inorganic and organic origin (ammonium salts) of *exo*-16,17-dihydrogibberellin A₅ and 2,3-dehydro-*exo*-16,17-dihydrogibberellin A₉ derivatives, their use in agriculture, and preparations containing these compounds. The compounds of the invention are particularly suitable for agricultural and biotechnological uses, and their great advantage is increased efficiency, water solubility and bioavailability, which leads to higher crop yields.

## Description

### Field of Art

The invention relates to single and double salts of inorganic and organic origin (ammonium salts) of derivatives of *exo*-16,17-dihydro gibberellin A₅ and *exo*-2,3-dehydro-16,17-dihydro gibberellin A₉, their use in agriculture, and to preparations containing these derivatives.

### Background Art

Gibberellins (GAs) are the oldest known plant hormones belonging to the group of diterpenoid acids, which are synthesized via the terpenoid pathway in plastids and subsequently modified in the endoplasmic reticulum and cytosol. In terms of their function, they regulate various developmental processes, including stem elongation, germination, dormancy, flowering, flower development and leaf and fruit senescence. Currently, 136 endogenous gibberellins are known in plants, fungi and bacteria, but only 4 of them are biologically active (Sponsel in Annual Plant Reviews, 2026, Vol. 49, Chapter 1). From a structural point of view, all gibberellins are derived from the ent-gibberellane skeleton, but their biosynthesis is based on *ent*-kaurene. Gibberellins are labelled in ascending chronological order according to their date of identification, from GA₁ to GAn (Sponsel and Hedden in Plant Hormones: Biosynthesis, Signal Transduction, Action, 2010, p. 63-94). Because gibberellins regulate various developmental processes, including stem elongation, germination, dormancy, flowering, flower development, and leaf and fruit senescence, several classes of growth regulators (inhibitors) of GA biosynthesis are currently used in agriculture.

Currently, three different classes of inhibitors of GA biosynthesis are known in higher plants, each of which inhibits GA biosynthesis at three different stages: class (1) inhibits the biosynthesis of ent-kaurene in proplastids; class (2) inhibits the conversion of *ent*-kaurene to GA₁₂ by cytochrome P450-containing microsomal monooxygenases; class (3) inhibits the biosynthesis of gibberellins containing 20 carbons in the chain (derived from GA₁₂) to gibberellins with 19 carbons in the structure. A well-known example of class 2 retarder inhibitors is chlormequat chloride (CCC), which is widely used as a wheat lodging control agent. Several heterocyclic nitrogen-containing compounds, including ancymidol, paclobutrazol, and tetcyclase, are effective inhibitors of *ent*-kaurene oxidase. A third group of inhibitors are acylcyclohexanedione derivatives, such as prohexadione, which target 2-oxoglutarate-dependent dioxygenases. Although these compounds are efficient, there is a growing consumer concern about the potential harmful side effects of these chemicals. This type of demand has led to a greater emphasis on the development of natural products that are less likely to cause adverse side effects.

Recently, phytohormones, or rather the regulation of their metabolism, biosynthesis or signaling, has become an increasingly important and useful method in the search for a way to increase crop yield and productivity. From this point of view, however, it is important to develop new types of growth modulators so that their action affects only the last steps of biosynthesis and does not interact with the previous stages. The ideal candidate turned out to be a derivative of gibberellin A₃ (13-O-acetate-16,17-dihydro-GA₅), which showed high inhibitory activity against the growth activity of plants belonging to the grass group (reduction of lodging and increase in grain yield), which include cereals such as corn, wheat and rice (Beck et al. Can. J. Chem. 2004, 82, 293-300). However, the disadvantage of this substance is its low solubility in aqueous solutions, and therefore reduced bioavailability. There is therefore still a need to provide growth modulators based on natural substances for use in agriculture which, while having sufficiently interesting efficacy, have suitable application properties.

### Disclosure of the Invention

The present invention provides gibberellin salts of general formula **I** 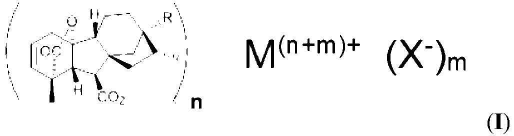
wherein n+m is 1 or 2;
n is 1 or 2;
m is 0 or 1;
wherein
when n+m is 1, then m is 0 and n is 1, and M⁺ is selected from the group of monovalent alkali metals, monovalent transition metals, and ammonium salts of the general formula N⁺HR'₃, wherein R' are independently selected from C1-C4 alkyls and C1-C4 hydroxyalkyls;
when n+m is 2, then m is 0 or 1 and n is 1 or 2, and M²⁺ is selected from the group of divalent alkaline earth metals and divalent transition metals;
R is OAc (i.e. -O-C(O)-CH₃) or H; and
X⁻ (if present) is a halide anion or an anion derived from a nitrogen-containing acid.

When n+m is 1, m = 0 and n = 1, the formula of such compounds can be expressed as formula **II**:

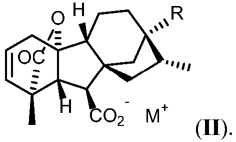

In a preferred embodiment, M⁺ is selected from the group Li⁺, Na⁺, K⁺, Cu⁺, Ag⁺, and ammonium salts of the general formula N⁺HR'₃, wherein R' are independently selected from methyl, ethyl, propyl, butyl, 2-hydroxyeth-1-yl, 2-hydroxyprop-1-yl.

When n+m is 2, m = 0 and n = 2, the formula of such compounds can be expressed as formula III:,

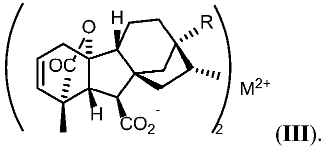

In a preferred embodiment, M²⁺ is selected from the group Mg²⁺, Ca²⁺, Sr²⁺, Ba²⁺, Zn²⁺, Cu²⁺, Mn²⁺, Fe²⁺.

When n+m is 2, m = 1 and n = 1, the formula of such compounds (mixed salts) can be expressed as formula **IV:**

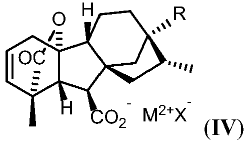

In a preferred embodiment, M²⁺ is selected from the group of Mg²⁺, Ca²⁺, Sr²⁺, Ba²⁺, Zn²⁺, Cu²⁺, Mn²⁺, Fe²⁺.

X⁻ is preferably selected from the group F⁻, Cl⁻, Br⁻, I⁻, NO₂⁻, NO₃⁻.

The salts of general formula **I** can be classified as salts of 16,17-dihydrogibberellin-A₅-13-acetate and 2,3-dehydro-16,17-dihydrogibberellin-A₉.

Preferred compounds of general formula **I,** falling under formula **II,** are selected from the group comprising:
lithium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, sodium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, copper(I) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, silver(I) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, trimethylammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, triethylammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, tripropylammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, tris(3-hydroxypropyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, lithium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, sodium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, potassium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[alazulene-10-carboxylate, copper(I) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, silver(I) (1*R*,4a*R*,4b*R*,7*R*,8*R*,9a*S*,10*S*,10aR)-1,8-dimethyl-13-oxo-1,4,4b,5,6,7,8,9,10,10a-decahydro-4a,1-(epoxymethano)-7,9a-methanobenzo[a]azulene-10-carboxylate, trimethylammonium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, triethylammonium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, tripropylammonium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, tris(2-hydroxyethyl)ammonium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, tris(3-hydroxypropyl)ammonium (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate,

Preferred compounds of general formula **I,** falling under formula **IV,** are selected from the group comprising:
Magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, strontium(II) (1*R*,4a*R*,4b*R*,7*S*,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, barium(II) (1*R*,4a*R*,4b*R*,7*S*,8*R*,9a*S*,10*S*,10a*R)*-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate fluoride, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate bromide, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate iodide, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrite, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate nitrate, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodidie, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, zinc(II) (1*R*,4a*R,*4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate fluoride, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate chloride, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate bromide, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate iodide, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrite, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate nitrate;

Preferred compounds of general formula I, falling under general formula **III,** are selected from the group comprising:
magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, calcium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, strontium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, barium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, zinc(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, copper(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, manganese(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, iron(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate, magnesium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, calcium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, strontium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, barium(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, zinc(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, copper(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, manganese(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate, iron(II) (1*R*,4a*R*,4b*R*,7*R*,8*R*,9a*S*,10*S*,10a*R*)-1,8-dimethyl-13-oxo-1,4,4b,5,6,7,8,9,10,10a-decahydro-4a,1-(epoxymethano)-7,9a-methanobenzo[a]azulene-10-carboxylate.

Particularly preferred compounds according to the invention are compounds of general formula I selected from the group containing: potassium (1*R*,4a*R*,4b*R*,7*S,*8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate; tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate; magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate. Structural formulas of these substances are given herein below.

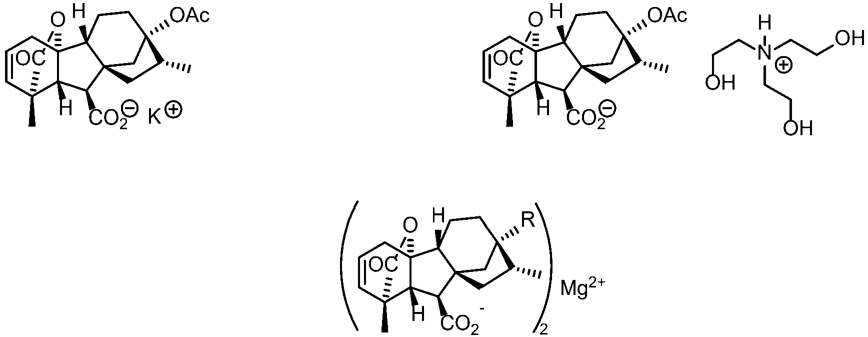

Compounds of formula I, where n = 1 and m = 0, are generally prepared by reacting a carboxylic acid corresponding to the anion with a base or a corresponding amine. In particular, the copper(I) salt is preferably prepared from the silver salt of general formula **I,** where n = 1 and m = 0, by reaction with a solution of CuCl.

Compounds of formula **I,** where n = 1 and m = 1, are generally prepared from a silver salt of general formula **I,** where n = 1 and m = 0, by reaction with MX₂, wherein M is a divalent metal cation (e.g., Mg, Ca, Sr, Ba, Zn, Cu, Mn, or Fe), and X is a halide anion (F, Cl, Br, or I). Compounds of formula **I,** where n = 1 and m = 1, in which X is NO₂ or NO₃, are prepared by reacting compounds of general formula **I,** wherein X = Cl, with AgNO₂ or AgNO₃, preferably in acetonitrile.

Compounds of formula **I,** where n = 2 and m = 0, are generally prepared by reacting a carboxylic acid corresponding to the anion with a base, or by reacting a silver salt of the anion of formula **I,** where n = 1 and m = 0, with a corresponding chloride of a divalent metal, preferably in acetonitrile.

The compounds of general formula **I** exhibit improved physicochemical properties, particularly better solubility in polar solvents including water, increased stability, and enhanced bioavailability. They also show improved efficiency, even in tests which remove the effect of increased solubility.

The compounds of general formula **I** according to the present invention reduce the elongation of cereal stems during the vegetative period and thus increase the yield of these crops. Their great advantage, which contributes to the increase of their biological activities, is increased solubility in water and bioavailability. The compounds according to the present invention (and preparations containing them) have minimal or zero toxicity. This allows their use in a wide range of applications.

The present invention further relates to the use of compounds of general formula **I** for increasing yield of crops, in particular cereals (wheat, barley, rice, maize, rye, oat, sorghum, and related species), beet (sugar beet and fodder beet); pomes, drupes and soft fruits (apples, pears, plums, peaches, almonds, cherries, strawberries and blackberries); leguminous plants (beans, lentils, peas, soybeans); oil plants (rape, mustard, poppy, olives, sunflowers, coconut, *Ricinus,* cocoa beans, groundnuts); cucumber plants (pumpkins, cucumbers, melons); fiber plants (cotton, flax, hemp, jute); citrus fruit (oranges, lemons, grapefruit, mandarins); vegetables (spinach, cinnamomum, camphor) or plants such as tobacco, nuts, eggplants, sugar cane, tea, vine grapes, hops, bananas and natural rubber and medicinal plants, as well as ornamentals. Crops include those which have been rendered tolerant towards classes of growth factors by conventional breeding methods or genetic engineering methods.

The present invention further provides agricultural and/or biotechnological compositions comprising at least one compound of general formula **I,** and an acceptable carrier and/or solvent. The compounds of general formula **I** are used in unmodified form or, preferably, together with excipients conventionally employed in the art of preparations. To this end they are conveniently formulated as concentrates of active compounds as well as suspensions and dispersions, preferably isotonic water solutions, suspensions and dispersions, diluted emulsions, soluble powders, dusts, granulates, creams, gels, oil suspensions and also encapsulations, e.g., polymeric substances. As with the type of the preparation, the methods of application, such as spraying, atomizing, dusting, scattering, coating or pouring, are chosen in accordance with the intended objectives and the prevailing circumstances. The preparations may be sterilized and/or contain further excipients of neutral nature such as preservatives, stabilizers, wetting agents or emulsifiers, solubilizing agents, as well as fertilizers, micronutrient donors or other formulations for obtaining special effects.

### PREPARATIONS

Preparations comprising a compound of general formula I (active ingredient) and, where appropriate, one or more solid or liquid auxiliaries (excipients), are prepared in a known manner, e.g. by mixing and/or grinding the active ingredients with the excipients, e.g. solvents or solid carriers. In addition, surface-active compounds (surfactants) may also be used in the preparations.

Depending on the nature of the compound of general formula **I** to be formulated, suitable surface-active compounds are non-ionic, cationic and/or anionic surfactants and surfactant mixtures having good emulsifying, dispersing and wetting properties.

Examples of suitable anionic, non-ionic and cationic surfactants are listed, for example, in WO 97/34485.

Also suitable in the preparation of the compositions containing compound of general formula I according to the invention are the surfactants conventionally used in formulation technology, which are described, *inter alia,* in "McCutcheon's Detergents and Emulsifiers Annual" MC Publishing Corp., Ridgewood New Jersey, 1981; Stache, H., "Tensid-Taschenbuch", Carl Hanser Verlag, Munich, 1981; and M. and J.Ash, "Encyclopedia of Surfactants", Vol.1-3, Chemical Publishing Co., New York, 1980-81.

The formulation of the preparation usually contains from 0.1 to 99 %, especially 0.1 to 95% (w/w) of active ingredient of cytokinin mesylate and from 0.1 to 25 % by weight of a surfactant.

While commercial products are usually formulated as concentrates, the end user will normally employ diluted formulations. The compositions may also comprise further ingredients, such as stabilizers, e.g. vegetable oils or epoxidized vegetable oils (epoxidized palm oil, rapeseed oil or soybean oil), antifoams, e.g. silicone oil, preservatives, stabilizers, wetting agents or emulsifiers, viscosity factors, binders, tackifiers, and also fertilizers or other active ingredients. Preferred formulations have especially the following compositions: (% = percent by weight).

### Emulsifiable concentrates:

| | |
|---|---|
| active ingredient: | 1 to 90 %, preferably 5 to 20 % |
| surfactant: | 1 to 30 %, preferably 10 to 20 % |
| liquid carrier: | 5 to 94 %, preferably 70 to 85 % |

### Dusts:

| | |
|---|---|
| active ingredient: | 0.1 to 10 %, preferably 0.1 to 5 % |
| solid carrier: | 99.9 to 90 %, preferably 99.9 to 95 % |

### Suspension concentrates:

| | |
|---|---|
| active ingredient: | 5 to 75 %, preferably 10 to 50 % |
| water: | 94 to 24 %, preferably 88 to 30 % |
| surfactant: | 1 to 40 %, preferably 2 to 30 % |

### Wettable powders:

| | |
|---|---|
| active ingredient: | 0.5 to 90 %, preferably 1 to 80 % |
| surfactant: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.9 to 70 %, preferably 99.9 to 85 % |

Different methods and technologies are used for the use of compounds of general formula **I** or compositions containing them, such as the following:
The compositions may also comprise further ingredients, such as stabilizers, e.g. vegetable oils or epoxidized vegetable oils (epoxidized coconut oil, rapeseed oil or soybean oil), anti-foams, e.g. silicone oil, preservatives, viscosity regulators, binders, tackifiers, and also fertilizers or other active ingredients. For the use of the compounds of general formula **I,** or of compositions comprising these compounds, various methods and techniques come into consideration, such as, for example, the following:

### I) Application as a tank mixture by spraying

A liquid formulation of the active ingredient, optionally together with another growth regulator is sprayed to the plants, the rate of application of the active ingredient being from 0.05 to 0.5 kg per hectare. Such tank mixtures are applied before or after sowing, in particular to emerged plants (foliar application) in the amount of the liquid preparation of 100-600 l/ha, depending on the crop.

### II) Controlled release of active ingredients

The compound(s) of formula **I** are applied in solution to mineral granule carriers or polymerized granules (urea/formaldehyde) and dried. If desired, it a coating may be applied that allows the active ingredient to be released in metered amounts over a specific period of time (coated granules).

### Brief description of drawings

Fig. 1: Height of winter wheat (cm) after application of the control substance Anti-GIB2 in BBCH 31-32 (2024)
Fig. 2: Height of winter wheat (cm) after application of Anti-GIB2-K salt in BBCH 31-32 (2024)
Fig. 3: Height of winter wheat (cm) after application of Anti-GIB2-N salt in BBCH 31-32 (2024)
Fig. 4: Height of winter wheat (cm) after application of Anti-GIB2 salt in BBCH 33-34 (2024)
Fig. 5: Height of winter wheat (cm) after application of Anti-GIB2-K salt in BBCH 33-34 (2024)
Fig. 6: Height of winter wheat (cm) after application of Anti-GIB2-N salt in BBCH 33-34 (2024)

### Examples

The following examples serve to illustrate the invention without limiting its scope. Unless stated otherwise, all percentages and similar quantities are based on weight.

Starting materials may be obtained from commercial sources (Sigma, Aldrich, Fluka, Merck, etc.) or may be prepared according to the procedures described below.

Thin-layer chromatography (TLC) was performed on Silica 60 F254 plates (Merck) using a CHCl₃/MeOH mixture as the mobile phase. Spots corresponding to individual substances were detected using UV light (254 and 365 nm) or a solution containing 6% vanillin in absolute EtOH with 1% H₂SO₄. Purification by column chromatography was carried out on Davisil 40-63-micron sorbent (Grace Davison). Elemental analysis was performed using a Flash EA 1112 analyzer (Thermo Scientific). Chromatographic purity and molecular weight of the prepared compounds were determined using an Alliance 2695 separation module (Waters) connected in parallel to a PDA 996 diode array detector (Waters) and a Q-Tof micro (Waters) benchtop quadrupole orthogonal acceleration time-of-flight tandem mass spectrometer. Samples were dissolved in DMSO and subsequently diluted to a concentration of 10 µg/ml directly in the liquid mixture used as the initial mobile phase. Samples (10 µl) were injected onto a reversed-phase column (RP column) Symmetry C18 (150 mm × 2.1 mm × 3.5 µm, Waters) and separated at a flow rate of 0.2 ml/min using the following binary gradient:
- 0 min, 10% B;
- 0-24 min, linear gradient to 90% B;
- followed by 10 min isocratic elution with 90% B.

After completion of the gradient, the column was re-equilibrated to the initial conditions. A 15 mM solution of formic acid was adjusted to pH 4.0 using ammonium hydroxide, and this mixture was used as solution A; methanol was used to dissolve the organic component (solvent B). The eluent was introduced into the DAD (scanning range 210-400 nm, with 1.2 nm resolution) and the ESI source (source temperature 110 °C, capillary voltage +3.0 kV, cone voltage +20 V, desolvation temperature 250 °C). Nitrogen was used as the desolvation gas (500 l/h) and cone gas (50 l/h). Data were acquired in positive ionization mode (ESI⁺) over the range of 50-1000 m/z.

¹H and ¹³C NMR spectra were measured using a Jeol ECA-500 NMR spectrometer operating at 500 MHz (¹H) and 126 MHz (¹³C), or a Jeol ECA-400 NMR spectrometer operating at 400 MHz (¹H) and 100 MHz (¹³C). Samples for measurement were prepared by dissolving the substance in DMSO-d₆, and the measured chemical shifts were calibrated against the peak of residual non-deuterated solvent DMSO-ds (2.50 ppm for proton) or against the solvent DMSO-d₆ (39.52 ppm for carbon).

### Characterization of Crystalline Salts of Formula I by ¹H Nuclear Magnetic Resonance (NMR)

¹H Nuclear Magnetic Resonance (¹H NMR) was performed using a JEOL 500 SS or JEOL 400 instrument operating at a temperature of 300 K and a frequency of 500.13 MHz or 400.1 MHz. Samples were prepared by dissolving the substances in DMSO-d₆. Tetramethylsilane (TMS) was used as the internal standard. Chemical shift calibration was referenced to the residual solvent peak of ¹H DMSO-d₆ at 2.50 ppm. Each sample was prepared at a concentration of approximately 5 mg.

The chemical shifts identified in the spectra of the crystalline salts (see individual examples above) reflect the original structure corresponding to the carboxylic acid ((1*R*,4a*R*,4b*R*,7*S*,8*R*,9a*S*,10*S*,10a*R*)-7-acetoxy-1,8-dimethyl-13-oxo-1,4,4b,5,6,7,8,9,10, l0a-decahydro-4a,1-(epoxymethano)-7,9a-methanobenzo[a]azulene-10-carboxylic acid), but compared to its spectrum, the individual shifts and splitting patterns of the substances are slightly altered, which corresponds to the presence of the cation in the measured sample.

The characteristic peaks of the starting carboxylic acid are as follows:
¹H NMR (500 MHz, DMSO-d₆) δ (ppm): 5.97 - 5.73 (m, 1H), 5.66 (dt, *J* = 9.3, 2.1 Hz, 1H), 2.85 (d, *J* = 10.2 Hz, 1H), 2.71 (d, *J* = 10.3 Hz, 1H), 2.53 (d, *J* = 10.4 Hz, 1H), 2.50 (h, *J =* 2.5 Hz, 2H), 2.32 - 2.19 (m, 1H), 2.15 - 2.08 (m, 1H), 2.06 (dd, *J* = 10.7, 2.9 Hz, 2H), 1.97 (dd, *J* = 11.7, 6.1 Hz, 1H), 1.94 (s, 3H), 1.88 - 1.81 (m, 2H), 1.79 (d, *J* = 10.9 Hz, 1H), 1.67 (dtd, *J =* 13.3, 6.4, 2.6 Hz, 1H), 1.18 (dd, *J* = 12.6, 6.0 Hz, 1H), 1.14 (s, 3H), 0.86 (d, *J* = 7.0 Hz, 3H).

¹³C NMR (101 MHz, DMSO-*D*₆) δ 177.58, 173.64, 170.24, 132.02, 128.93, 92.09, 85.21, 54.95, 53.74, 52.16, 49.55, 48.13, 44.45, 41.12, 37.56, 35.13, 21.89, 19.24, 17.55, 15.70.

### Example 1: Preparation of potassium(I) (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (1)

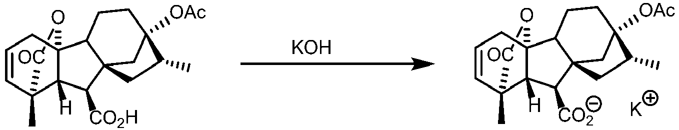

(1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylic acid (1.04 g; 2.4 mmol) was dissolved in methanol (31 ml) at room temperature (RT), and an aqueous solution of KOH (0.02 M, 60 ml) was slowly added. The resulting reaction mixture was stirred for 30 minutes, and the pH of the solution was monitored and adjusted using KOH solution to a value above 7.
Yield: 99 %; purity (HPLC-UV/VIS): 98 %+; MS (ESI⁻): [M]⁻= 373.4

¹H NMR (400 MHz, DMSO-d₆) δ (ppm): 5.77 (d, *J* = 9.1 Hz, 1H), 5.62 (d, *J =* 9.2 Hz, 1H), 2.60 (d, *J* = 9.9 Hz, 1H), 2.38 (s, 2H), 2.09 (d, *J =* 8.9 Hz, 2H), 1.98 (s, 2H), 1.94 (s, 3H), 1.74 (q, *J* = 10.4 Hz, 4H), 1.57 (d, *J* = 9.5 Hz, 2H), 1.35 (dd, *J* = 12.3, 6.0 Hz, 1H), 1.07 (s, 3H), 0.79 (d, *J* = 6.7 Hz, 3H). ¹³C NMR (101 MHz, DMSO-*d*₆) δ (ppm): 178.39, 169.77, 132.72, 127.89, 91.98, 85.48, 55.67, 53.57, 48.92, 47.96, 44.31, 40.61, 37.35, 34.95, 21.48, 18.79, 17.26, 15.41.

### Example 2: Preparation of tris(2-hydroxyethyl)ammonium (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (2)

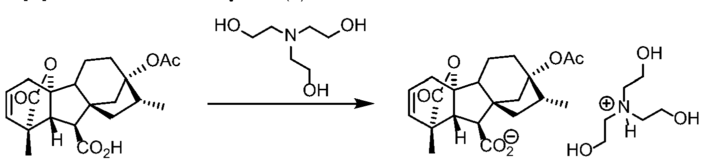

**(1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-**4a,1-(epoxymethano)-7,9a-methanobenzo[a]azulene-10-carboxylic acid (300 mg; 0.84 mmol) was dissolved in methanol (15 ml) at room temperature, and an aqueous solution of 2,2',2"-nitrilotris(ethan-1-ol) (113 µL, 0.84 mmol) in water (28 ml) was slowly added. The resulting reaction mixture was stirred for 6 hours at 50 °C (during which the solution became clear). The reaction mixture was then cooled to room temperature, and the solvents were evaporated using a rotary vacuum evaporator, yielding a viscous syrup. The syrup was dissolved in 10 ml of water, and the excess water was removed by lyophilization, resulting in a solid crystalline powder (421 mg).
Yield: 99 %; purity (HPLC-UV/VIS): 98 %+; MS (ESI⁻): [M]⁻= 373.3

¹H NMR (500 MHz, DMSO-d₆) δ (ppm): 5.84 (dt, *J* = 9.3, 3.2 Hz, 1H), 5.67 (dt, *J* = 9.1, 2.1 Hz, 1H), 4.59 (broad s, 1H), 3.45 (t, *J =* 6.0 Hz, 6H), 3.37 (broad s, 3H), 2.67 - 2.63 (m, 6H), 2.61 (d, *J* = 10.3 Hz, 2H), 2.48 - 2.42 (m, 2H), 2.38 - 2.33 (m, 1H), 2.23 - 2.13 (m, 1H), 2.10 - 1.98 (m, 2H), 1.96 (s, 3H), 1.95 - 1.88 (m, 2H), 1.85 - 1.74 (m, 1H), 1.74 - 1.65 (m, 2H), 1.62 (dh, *J* = 10.9, 4.0, 3.2 Hz, 1H), 1.09 (s, 3H), 0.82 (d, *J* = 6.9 Hz, 3H).

¹³C NMR (126 MHz, DMSO-d₆) δ (ppm): 177.46, 169.77, 131.90, 128.30, 91.73, 84.98, 58.04, 56.55, 53.43, 47.78, 44.11, 40.70, 37.20, 34.79, 21.43, 18.78, 17.17, 15.30.

### Example 3: Preparation of silver(I) (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (3)

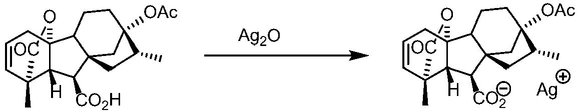

(1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylic acid (300 mg; 0.84 mmol) was dissolved in acetonitrile (15 ml) at room temperature, and Ag₂O (97 mg; 0.42 mmol) was added. The resulting reaction mixture was stirred for 6 hours at room temperature in the absence of light. The reaction mixture was then concentrated using a rotary vacuum evaporator, yielding a solid crystalline powder (402 mg).
Yield: 99 %; purity (HPLC-UV/VIS): 98 %+; MS (ESI⁻): [M]⁻= 373.4

¹H NMR (400 MHz, DMSO-d₆) δ (ppm): 5.80 (dt, *J =* 9.3, 3.2 Hz, 1H), 5.64 (d, *J =* 9.4 Hz, 1H), 2.67 (p, *J* = 1.9 Hz, 1H), 2.61 (d, *J* = 10.0 Hz, 2H), 2.42 (s, 2H), 2.21 (d, *J* = 10.4 Hz, 1H), 2.14 (d, *J* = 7.0 Hz, 1H), 2.01 (d, *J* = 8.8 Hz, 2H), 1.95 (s, 2H), 1.80 (s, 2H), 1.72 (dd, *J =* 24.6, 11.2 Hz, 1H), 1.66 - 1.53 (m, 2H), 1.41 (dd, *J* = 12.8, 6.0 Hz, 1H), 1.11 (s, 3H), 0.82 (d, *J* = 6.9 Hz, 3H).

¹³C NMR (126 MHz, DMSO-*D*₆) δ 177.66, 170.26, 132.06, 128.92, 92.13, 85.26, 59.16, 57.39, 53.76, 52.24, 49.54, 48.16, 44.47, 41.13, 37.59, 35.16, 21.91, 19.26, 17.58, 15.73.

### Example 4: Preparation of magnesium (II) (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate chloride (4)

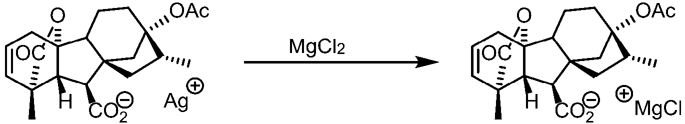

Silver(I) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (500 mg; 1.04 mmol) was dissolved in acetonitrile (15 ml) at room temperature, and MgCl₂ (99 mg; 1.04 mmol) was added. The resulting reaction mixture was stirred for 6 hours at room temperature (RT) in the absence of light. The reaction mixture was then filtered through filter paper, and the resulting filtrate was concentrated using a rotary vacuum evaporator, yielding a solid crystalline powder (401 mg).
Yield: 89 %; purity (HPLC-UV/VIS): 98 %+; MS (ESI⁻): [M]⁻= 373.4

¹H NMR (400 MHz, DMSO-d₆) δ (ppm): 5.80 (dt, *J =* 9.3, 3.2 Hz, 1H), 5.64 (d, *J =* 9.4 Hz, 1H), 2.61 (d, *J* = 10.0 Hz, 1H), 2.42 (s, 2H), 2.33 (p, *J =* 1.9 Hz, 1H), 2.21 (d, *J =* 10.4 Hz, 1H), 2.14 (d, *J* = 7.0 Hz, 1H), 2.01 (d, *J* = 8.8 Hz, 2H), 1.95 (s, 3H), 1.80 (s, 2H), 1.72 (dd, *J* = 24.6, 11.2 Hz, 1H), 1.65 - 1.53 (m, 2H), 1.41 (dd, *J* = 12.8, 6.0 Hz, 1H), 1.11 (s, 3H), 0.82 (d, *J* = 6.9 Hz, 3H).

¹³C NMR (101 MHz, DMSO-*d*₆) δ (ppm): 178.74, 170.23, 133.20, 128.36, 92.47, 85.97, 56.16, 54.01, 49.43, 48.43, 44.79, 41.03, 37.76, 35.43, 21.96, 19.26, 17.73, 15.88.

### Example 5: Preparation of magnesium (II) (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (5)

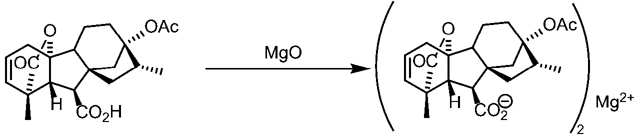

(1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylic acid (100 mg; 0.27 mmol) was dissolved in water (5 ml) and stirred at 50 °C for 20 minutes. MgO (5.4 mg; 0.134 mmol) in 1 ml of water was added, and the resulting mixture was stirred for 50 minutes at 75 °C. The resulting white crystalline solid was filtered, and the filtrate was washed with anhydrous methanol (2 × 5 ml). The final product was then dried for 6 hours at 110 °C.
Yield: 96 %; purity (HPLC-UV/VIS): 98 %+; MS (ESI⁻): [M]⁻= 373.4

¹H NMR (400 MHz, DMSO-*d*₆) δ (ppm): 5.80 (dt, *J=* 9.4, 3.2 Hz, 1H), 5.65 (d, *J* = 9.3 Hz, 1H), 2.70 - 2.58 (m, 1H), 2.41 (d, *J* = 10.1 Hz, 2H), 2.36 - 2.29 (m, 1H), 2.24 (s, 1H), 2.14 (q, *J* = 7.1 Hz, 1H), 2.01 (d, *J* = 7.9 Hz, 2H), 1.95 (s, 3H), 1.82 (d, *J* = 9.6 Hz, 2H), 1.75 - 1.65 (m, 1H), 1.65 - 1.53 (m, 2H), 1.46 - 1.33 (m, 1H), 1.11 (s, 3H), 0.82 (d, *J* = 6.8 Hz, 3H).

¹³C NMR (101 MHz, DMSO-d₆) δ (ppm): 177.95, 169.72, 132.43, 128.07, 91.81, 85.35, 55.38, 53.60, 53.33, 49.22, 47.81, 43.92, 40.43, 37.12, 34.93, 30.75, 21.45, 18.74, 17.19, 15.58.

### Example 6: In vitro cytotoxic activity of new derivatives on normal and tumor animal cells

Low cytotoxicity is essential for the use of these substances in agriculture. One of the parameters used as a basis for cytotoxic analysis is the metabolic activity of viable cells. For example, the microtiter assay, where Calcein AM is used, is now widespread as a method for quantifying cell proliferation and cytotoxicity. Only viable cells are detected by the assay. The amount of reduced Calcein AM corresponds to the number of viable cells in the culture.

The cell lines BJ (human foreskin fibroblasts), G361 (human malignant melanoma) and K562 (human bone marrow chronic myeloid leukemia) were used for routine screening of compounds. Cells were maintained in Nunc/Corning 80 cm2 plastic culture flasks and cultured in cell culture medium (DMEM with 5 g/L glucose, 2 mM glutamine, 100 U/mL penicillin, 100 x g/mL streptomycin, 10% fetal calf serum, and sodium bicarbonate).

Cell suspensions were prepared and diluted according to cell type and expected final cell density (2,500-30,000 cells per well based on cell growth characteristics), and 80 µl of cell suspension was pipetted into 96-well microtiter plates. Inoculum was stabilized by a 24-hour pre-incubation at 37 °C in an atmosphere of 5% CO₂. Individual concentrations of test substances were added at time zero as 20 µl aliquots to the wells of the microtiter plates. Typically, compounds were diluted to six concentrations in a four-fold dilution series. In routine testing, the highest concentration in the well was 166.7 µM, changes in this concentration depended on the substance. All concentrations were tested in triplicate. Incubation of cells with tested derivatives lasted for 72 hours at 37 °C, 100% humidity and in an atmosphere of 5% CO₂. At the end of the incubation period, cells were analyzed after addition of Calcein AM solution (Molecular Probes) and incubation continued for another 1 hour. Fluorescence (FD) was measured using a Labsystem FIA reader Fluorskan Ascent (Microsystems). Tumor cell survival (TCS) was calculated according to the following relationship: IC₅₀ = (FD_{well with derivative} /FD_{control well}) x 100%. The IC₅₀ value, which corresponds to the concentration of the substance at which 50% of the tumor cells are killed, was calculated from the obtained dose curves.

Zero cytotoxicity is a basic prerequisite for the use of these substances in agricultural applications. To evaluate the antitumor activity, the toxicity of the new derivatives was tested on panels containing cell lines of different histogenetic and species origin (Tab. 1). We show here that the same activities were found in all tested tumor cell lines, as well as in non-malignant cells, e.g. BJ fibroblasts. As shown in Table 1, the IC₅₀ for cancer cell lines as well as BJ fibroblasts was always higher than 50 µM. The prepared salts show no toxicity towards normal and tumor cells at concentrations around 50 µM and are therefore very suitable for agricultural applications.

**Table 1: Cytotoxicity of salts for various normal and cancer cell lines tested/IC₅₀ (µmol/l)**

| **Salt** | **CEM** | **BJ** | **MCF7** | **G-361** |
|---|---|---|---|---|
| **1** | >50 | >50 | >50 | >50 |
| **2** | >50 | >50 | >50 | >50 |
| **4** | >50 | >50 | >50 | >50 |
| **5** | >50 | >50 | >50 | >50 |

### Example 7: Comparison of the effect of selected prepared salts with the substance 16,17-dihydro-GA₅ based on competition experiments

To evaluate the inhibition efficiency of the prepared salts on the biosynthesis of gibberellin A4 (bioactive), a competitive experiment based on GA₃-oxidases was used, which, within the biosynthesis of gibberellins, enable the transformation of gibberellin A9 (non-bioactive) to A4 (bioactive). In this test, the efficiency of the transformation of gibberellin A₉ to A₄ was evaluated both in the absence of inhibitors and in the presence of 16,17-dihydro-GA₅ (positive control) and newly prepared salts at various concentrations. The inhibition efficiency was then related to the ratio of unreacted substrate to formed GA₄, where 0 means no inhibition (all substrate (GA₉) was transformed to GA₄) and K.I. complete inhibition of GA₄ formation (GA₄ formation was not detected during the experiment). The results are contained in Table 2.

The enzymatic assay for comparing the effect of inhibitors was performed according to the standard protocol (MacMillan et al. Plant Physiol. 1997, 113, 1369-1377) with minor modifications. The reaction mixture contained 100 mM Tris-HCl pH 7.2, 4 mM DTT, 4 mM ketoglutarate, 4 mM ascorbic acid, 500 µM FeSO₄, 2 mg/ml BSA, 0.1 mg/ml catalase, 200 nM recombinant TaGA3ox2 and 1 µM GA₉ (substrate). The enzymatic reaction was carried out for 30 min at 30 °C and was stopped by the addition of methanol and acetic acid to a final concentration of 33% and 3.3%, respectively. The ratio of the amount of substrate (GA9) and the resulting product (GA₄) in the samples was subsequently determined by the UHPLC MS (Waters) method according to Urbanová et al. (Talanta 2013, 112, 85-94) in single ion monitoring mode. Standards (1 µM GA₉ and GA₄), reactions without inhibitor (negative control), reactions inhibited by 16,17-dihydro-GA₅ (a positive control) and reactions with three salts of 16,17-dihydro-GA₅-13-acetate were compared. All reactions were performed in triplicate and inhibitors, including positive control, were tested at concentrations of 2, 10 and 50 µM.

The enzyme was obtained from the coding part of the wheat gene TaGA3ox2 with a codon optimization (GenScript), which was cloned into the pET32b vector and expressed in T7 competent *Escherichia coli* cells (NEB). The bacterial culture was induced in the exponential growth phase with 0.5 mM IPTG and further cultivated for 16 h at 20 °C. After centrifugation, the cells were resuspended in 100 mM Tris-HCl buffer pH 7.2 containing 100 mM NaCl, 5% glycerol and 2 mM DTT and lysed on a homogenizer (Constant Systems) at a pressure of 27 kPSI. Purification of the His-tag carrying TaGA3ox2 protein was performed on HisPur (Thermo Fisher Scientific) spin columns with cobalt resin and Amicon (Millipore) with a molecular cut-off of up to 30 kDa.

**Table 2: Activity of salts in a competitive inhibition experiment against GA3-ox2 at various concentrations.**

| | **GA₉ (peak area)** | **GA₄ (peak area)** | **GA₉ / GA₄ ratio** |
|---|---|---|---|
| **Negative control** (H₂O) | n.d.^{a} | 19514 ± 530 | 0 |

| **Positive control (solution in H₂O)** | | | |
|---|---|---|---|
| **16,17-diH-GA₅** (2 µM) | 3686 ± 188 | 13946 ± 113 | 0,3 |
| **16,17-diH-GA₅** (10 µM) | 12197 ± 740 | 4604 ± 34 | 2,7 |
| **16,17-diH-GAs** (50 µM) | 16980 ± 343 | 1143 ± 216 | 14,9 |

| **Salt (solution in H₂O)** | | | |
|---|---|---|---|
| 1 (2 µM) | 9310 ± 260 | 5308 ± 86 | 1,8 |
| **1** (10 µM) | 13457 ± 974 | 974 ± 12 | 13,8 |
| **1** (50 µM) | 15525 ± 557 | n.d. | >99 |
| **2** (2 µM) | 10534 ± 2044 | 3669 ± 467 | 2,9 |
| **2** (10 µM) | 13224 ± 652 | 626 ± 67 | 21,1 |
| **2** (50 µM) | 13780 ± 708 | n.d.^{a} | **K.I.^{b}** |
| **5** (2 µM) | 11745 ± 632 | 2587 ± 191 | 4,5 |
| **5** (10 µM) | 12431 ± 1307 | 559 ± 50 | 22,2 |
| **5** (50 µM) | 13351 ± 496 | n.d.^{a} | **K.I.^{b}** |

| | | | |
|---|---|---|---|
| ^{a} below detection limit; ^{b} complete inhibition. GA₄ formation was not detected. | | | |

### Example 8: Determination of the solubility of prepared salts using quantitative ¹H-nuclear magnetic resonance

¹H Nuclear magnetic resonance (¹H NMR) was performed on a JEOL 500 SS instrument operating at 300 K and 500.13 MHz. The solubility was determined by preparing a supersaturated solution in D₂0 of each compound **(16,17-diH-GA₅, 1,2 and 5).** The undissolved solid residue from the liquid phase was then separated by filtration (0.2 µm) and centrifugation (Eppendorf 5415R, 5 min, 8000 g) to obtain saturated solutions. 560 µL of the solution was taken from this sample and 2 µL of dimethyl sulfone (Me₂SO₄) was added as an internal standard. The ¹H NMR spectrum of the thus prepared sample was measured and the resulting concentration of the individual substances was then determined from the following relationship, where C_{comp} = I_{comp}/ x N_{cal}/N_{comp} x C_{cal}; where C_{cal} is the molar concentration of the substance of interest (mmol/L), I_{comp} corresponds to the peak area from the ¹H NMR spectrum, N is the number of measured nuclei and C_{cal} is the concentration of the standard. For the standard (dimethyl sulfone), the values were determined for the characteristic peak of 3.2 ppm, where I_{cal} corresponded to the measured peak area, N_{cal} = 6, and the used concentration C_{cal} = 55 mM. For the measured substances, the characteristic peak at 5.66 ppm or its equivalent depending on the measured substance was selected, where I_{comp} corresponded to the measured peak area and N_{comp} = 1. The determined concentrations are in Table 3.

**Table 3: Determined maximum solubilities of salts 1,2 and 5 and 16,17-dihydro-13acetate-GA₅. Concentrations are given in mM.**

| **Compound** | **concentration** | **deviation** |
|---|---|---|
| **16,17-dihydro-13acetate-GA₅** | 2.25 | ±0.22 |
| **1** | 10.2 | ±0.3 |
| **2** | 11.3 | ±0.3 |
| **5** | 10.1 | ±0.5 |

### Example 9: Effect of anti-GIB2-K on grain yield and plant height of spring barley (var. Francin)

Spring barley (var. Francin) was treated in 2022-2023 at a location in Olomouc by spraying substances - labelled anti-GIB2-K (potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The sowing rate was 3.5 million germinating seeds. The experiment was based on 5 randomized replications for each variant; the control was the untreated variant. The experiment was conducted according to the GEP (Good Experimental Practice) methodology, the size of individual plots was 10 m². The application of the substances was carried out in the amount of 300 l/ha, in the growth phase BBCH 30-33 (beginning of stem elongation), BBCH 37-45 and BBCH 51. The concentration of the tested anti-GIB2-K substance in the spray solution was 500 µM. Trinexapac-ethyl and CCC (chlorocholine chloride) were used for comparison as positive controls. These are substances known from the prior art, which are used practically in agriculture as growth retardants. The results are summarized in Tables 4 and 5 below.

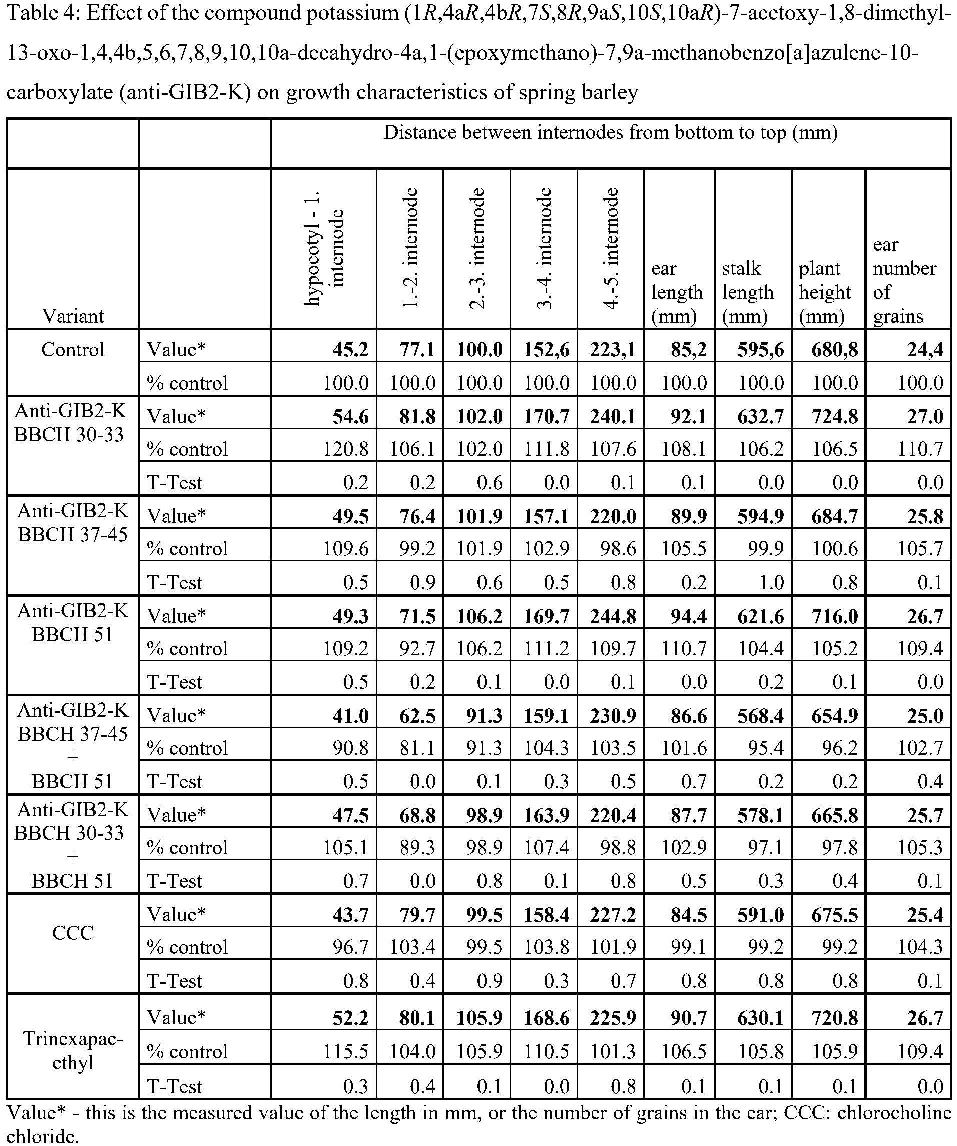

**Table. 5: Effect of the substance potassium (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (anti-GIB2-K) on yield results of spring barley. The application date is given in Table 4.**

| | Term of application | | | Yield | % control | T-test | Plant height | % control | T-test |
|---|---|---|---|---|---|---|---|---|---|
| Variant | BBCH 30-33 | BBCH 37-45 | BBCH 51 | (t/ha) | | | cm | | |
| Control | | | | **8.810** | 100.00 | | **65.167** | 100.000 | |
| Anti-GIB2-K | 500 µM | | | **9.566** | 108.58 | *0.182* | **68.000** | 104.348 | *0.094* |
| Anti-GIB2-K | | 500 µM | | **10.365** | 117.65 | *0.005* | **67.200** | 103.120 | *0.234* |
| Anti-GIB2-K | | | 500 µM | **9.809** | 111.33 | *0.035* | **61.600** | 94.527 | *0.060* |
| Anti-GIB2-K | | 500 µM | 500 µM | **10.577** | 120.05 | *0.012* | **57.267** | 87.877 | *0.000* |
| Anti-GIB2-K | 500 µM | | 500 µM | **10.260** | 116.46 | *0.008* | **60.583** | 92.967 | *0.043* |
| CCC | 1.0 l/ha | | | **10.325** | 117.19 | *0.003* | **67.333** | 103.325 | *0.263* |
| Trinexapac-ethyl | 0.4 l/ha | | | **10.073** | 114.34 | *0.045* | **69.600** | 106.803 | *0.023* |

| | | | | | | | | | |
|---|---|---|---|---|---|---|---|---|---|
| CCC: chlorcholine chlorid. | | | | | | | | | |

Foliar application of all tested derivatives and combinations increased yield (by 8.58 to 20.05% above the control level). Most combinations had even a stronger effect on yield than the positive controls Trinexapac-ethyl and CCC. The length of the stalk was shortened in a number of variants (especially later applications), which had a positive effect on less lodging of barley. The shortening of the stalk occurred mainly between the 1^{st}-3^{rd} internode, which also had an effect on the total length of the plants.

### Example 10: Effect of anti-GIB2-K on grain yield and growth characteristics of wheat plants

Winter wheat (var. Turandor) was treated in 2022-2023 at a location in Olomouc by spraying substance labelled anti-GIB2-K (potassium (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The aim of this test was to find the optimal dosage and also the method of application depending on the developmental stages of winter wheat. The sowing rate was 3.5 million germinating seeds. The experiment was based on 5 randomized repetitions for each variant; the control was the untreated variant. The experiment was conducted according to the GEP (Good Experimental Practice) methodology, the size of individual plots was 10 m². The application of substances was carried out in an amount of 300 l/ha, in the growth phase BBCH 30-33 (beginning of stem elongation), BBCH 37-45 and BBCH 51. The concentration of the tested substance anti-GIB2-K in the spray solution was 500 µM. Trinexapac-ethyl and CCC (chlorocholine chloride) were used for comparison as positive controls. These are substances known from the prior art, which are used practically in agriculture as growth retardants. The results are summarized in Table No. 6 and 7 below.

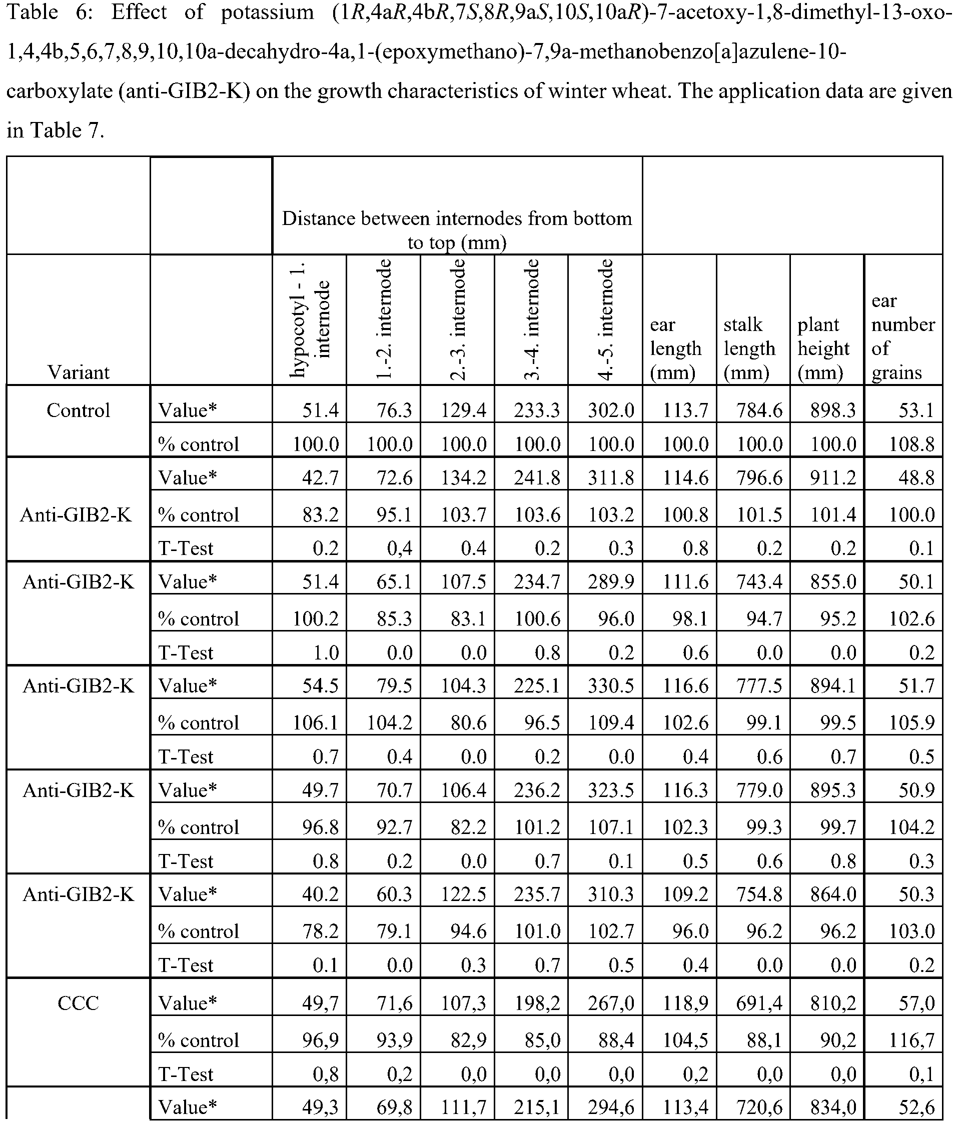

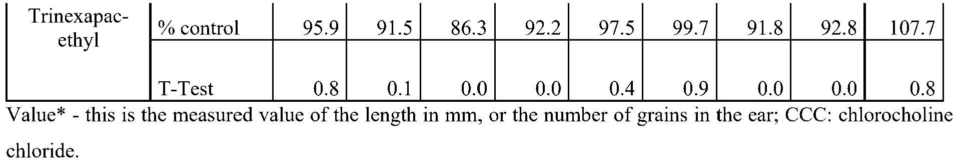

**Table 7: Effect of potassium (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate (anti-GIB2-K) on yield results of winter wheat.**

| **Comp** | Term of aplication | | | | | | | | Plant height 23.5.2022 | | Plant height 1.6.2022 | |
|---|---|---|---|---|---|---|---|---|---|---|---|---|
| | BBCH 30-33 | BBCH 37-45 | BBCH 51 | Yield t/ha | % CTRL | T-test | WTS (g) | % CTRL | Mean (cm) | % CTR L | Mean (cm) | % CTRL |
| **Control** | | | | **10.0** | **100.0** | | 52.2 | 100.0 | | | | |
| Anti-GIB2-K | 500 µM | | | **11.0** | **110.0** | *1.0* | 49.8 | 99.8 | **55.1** | 86.3 | **77.5** | 94.6 |
| Anti-GIB2-K | | 500 µM | | **10.9** | **110.9** | *0.9* | 49.8 | 99.8 | **53.1** | 83.2 | **80.8** | 98.5 |
| Anti-GIB2-K | | | 500 µM | **11.0** | **110.0** | *1.0* | 47.7 | 95.6 | **56.6** | 88.7 | **81.4** | 99.3 |
| Anti-GIB2-K | | 500 µM | 500 µM | **10.4** | **104.0** | *0.3* | 49.2 | 98.7 | **50.6** | 79.3 | **76.1** | 92.8 |
| Anti-GIB2-K | 500 µM | | 500 µM | **11.8** | **111.8** | *0.2* | 48.7 | 97.6 | **50.1** | 78.6 | **75.1** | 91.5 |
| CCC | 1.0 l/ha | | | **10.6** | **106.0** | *0.4* | 48.0 | 96.2 | **56.5** | 88.6 | **71.9** | 87.6 |
| Trinexa pac-ethyl | 0.4 l/ha | | | **11.0** | **110.0** | *1.0* | 49.4 | 99.1 | **54.7** | 85.7 | **72.3** | 88.1 |

| | | | | | | | | | | | | |
|---|---|---|---|---|---|---|---|---|---|---|---|---|
| WTS: weight of a thousand seeds; CTRL: control; CCC: chlorocholine chloride, | | | | | | | | | | | | |

Foliar application of winter wheat resulted in an increase in yield only in some variants, however, most variants after application of the derivative had a higher grain yield (94.6 - 107.2% for the control untreated variant) than preparations based on CCC and Trinexapac-ethyl (96.4, respectively 99.7% for the control untreated variant). With repeated application in the BBCH 37-39 + BBCH 51 and BBCH 30-33 + BBCH 51 phases, there was a significant shortening of the stalk to 92.8, respectively 91.5% for the untreated control. The stalk length was shortened in a number of variants (especially later applications), which had a positive impact on wheat lodging. The stalk shortening occurred mainly in the area of the 1^{st}-3^{rd} internodes.

### Example 11: Effect of anti-GIB2 salts compared to anti-GIB2 applied at BBCH 30-33 on wheat stalk elongation

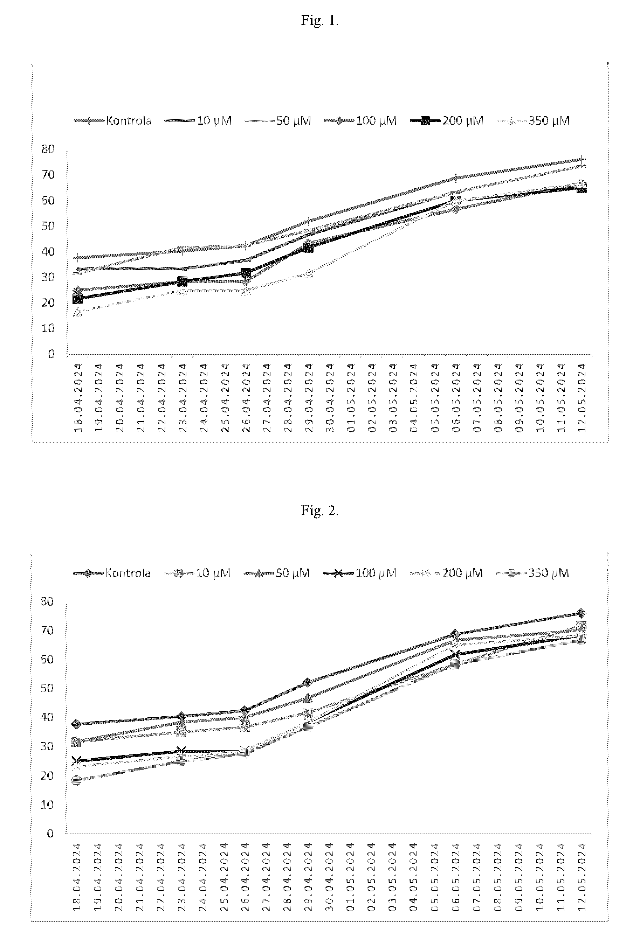

Winter wheat (var. Turandor) was treated in 2024 at a location in Olomouc by spraying with substances designated anti-GIB2-K (potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate) and anti-GIB 2-N (tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The starting substance Anti-GIB2 served as a control. The aim of this test was to find the optimal dosage depending on the developmental stages of winter wheat. The sowing volume was 3.5 million germinated seeds. The experiment was based on 5 randomized replicates for each variant; the control was the untreated variant. The experiment was carried out according to the GEP (Good Experimental Practice) methodology, the size of individual plots was 10 m2. The application of the substances was carried out in the amount of 300 l/ha, in the growth phase BBCH 30-33 (beginning of stem elongation - 1^{st} to 2^{nd} nodes palpable). The application concentrations were 10, 50, 100, 200 and 350 µM, the amount of spray solution was 300 l/ha. Subsequently, the height of the plants was evaluated at several dates and the length of individual internodes was analyzed. The results are summarized in Tables 8-10 and also in Figures No. 1-3.

**Table 8: Stem length of winter wheat (cm) after application of the control substance Anti-GIB2 (application in BBCH 31-32)**

| APPLICATION DATE | EVALUATION DATE | Control | 10 µM | 50 µM | 100 µM | 200 µM | 350 µM |
|---|---|---|---|---|---|---|---|
| 05.04.2024 | 18.04.2024 | 37.67 | 31.67 | 31.67 | 25.00 | 23.33 | 18.33 |
| 05.04.2024 | 23.04.2024 | 40.33 | 35.00 | 38.33 | 28.33 | 26.67 | 25.00 |
| 05.04.2024 | 26.04.2024 | 42.33 | 36.67 | 40.00 | 28.33 | 28.25 | 27.50 |
| 05.04.2024 | 29.04.2024 | 52.00 | 41.67 | 46.67 | 38.33 | 38.33 | 36.67 |
| 05.04.2024 | 06.05.2024 | 68.67 | 58.33 | 66.67 | 61.67 | 65.00 | 58.33 |
| 05.04.2024 | 12.05.2024 | 76.00 | 71.67 | 70.00 | 68.33 | 68.33 | 66.67 |

**Table 9: Stem length of winter wheat (cm) after application of Anti-GIB2-K salt (application in BBCH 31-32)**

| APPLICATION DATE | EVALUATION DATE | Control | 10 µM | 50 µM | 100 µM | 200µM | 350 µM |
|---|---|---|---|---|---|---|---|
| 05.04.2024 | 18.04.2024 | 37.67 | 31.67 | 31.67 | 25.00 | 23.33 | 18.33 |
| 05.04.2024 | 23.04.2024 | 40.33 | 35.00 | 38.33 | 28.33 | 26.67 | 24.50 |
| 05.04.2024 | 26.04.2024 | 42.33 | 36.67 | 40.00 | 28.33 | 28.25 | 27.20 |
| 05.04.2024 | 29.04.2024 | 52.00 | 41.67 | 46.67 | 38.33 | 38.33 | 36.67 |
| 05.04.2024 | 06.05.2024 | 68.67 | 58.33 | 66.67 | 61.67 | 65.00 | 58.03 |
| 05.04.2024 | 12.05.2024 | 76.00 | 71.67 | 70.00 | 68.33 | 68.33 | 62.12 |

**Table 10: Stem length of winter wheat (cm) after application of Anti-GIB2-N salts (application in BBCH 31-32)**

| APPLICATION DATE | EVALUATION DATE | Control | 10 µM | 50 µM | 100 µM | 200 µM | 350 µM |
|---|---|---|---|---|---|---|---|
| 05.04.2024 | 18.04.2024 | 37.67 | 28.33 | 30.00 | 28.33 | 23.33 | 16.67 |
| 05.04.2024 | 23.04.2024 | 40.33 | 35.00 | 36.67 | 31.67 | 26.67 | 25.00 |
| 05.04.2024 | 26.04.2024 | 42.33 | 36.67 | 40.00 | 36.67 | 28.33 | 27.50 |
| 05.04.2024 | 29.04.2024 | 52.00 | 43.33 | 48.33 | 41.67 | 38.33 | 36.33 |
| 05.04.2024 | 06.05.2024 | 68.67 | 58.33 | 61.67 | 65.00 | 63.33 | 57.74 |
| 05.04.2024 | 12.05.2024 | 76.00 | 70.00 | 70.00 | 70.00 | 68.33 | 62.00 |

The results of the application of Anti-GIB2-K and Anti-GIB2-N salts show the dependence of the effect of the derivative on the concentration of the substance in the spray solution. The highest concentration of 350 µM was the most effective. Application in the growth phase BBCH 31-32 did not have a very significant effect and no differences were observed between the applied salts. The final stem length was most affected by the concentration used, with the stem shortening effect only noticeable in the late stages of wheat growth.

### Example 12: Effect of anti-GIB2 salts compared to anti-GIB2 applied at BBCH 33-34 on wheat stalk elongation

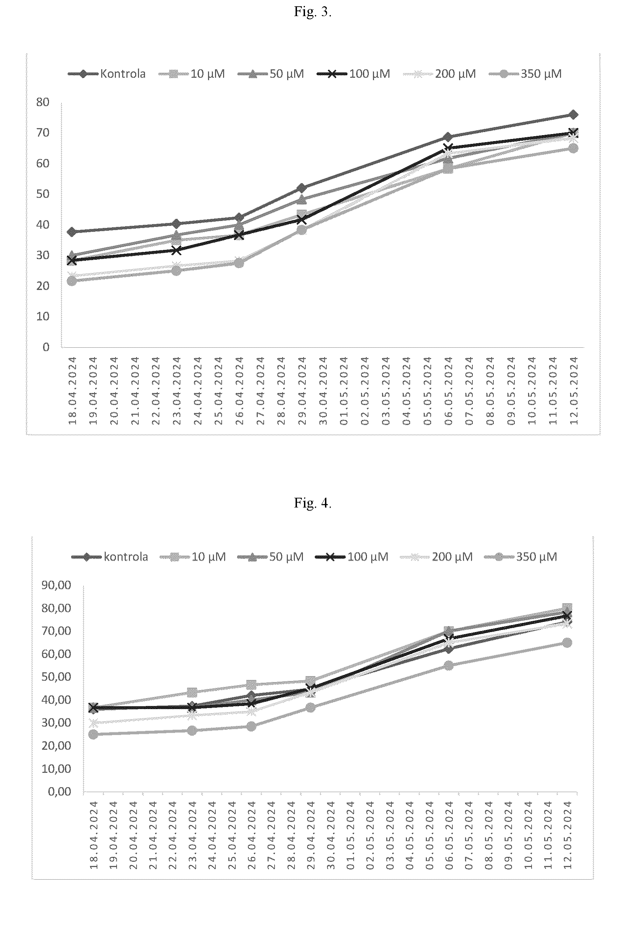

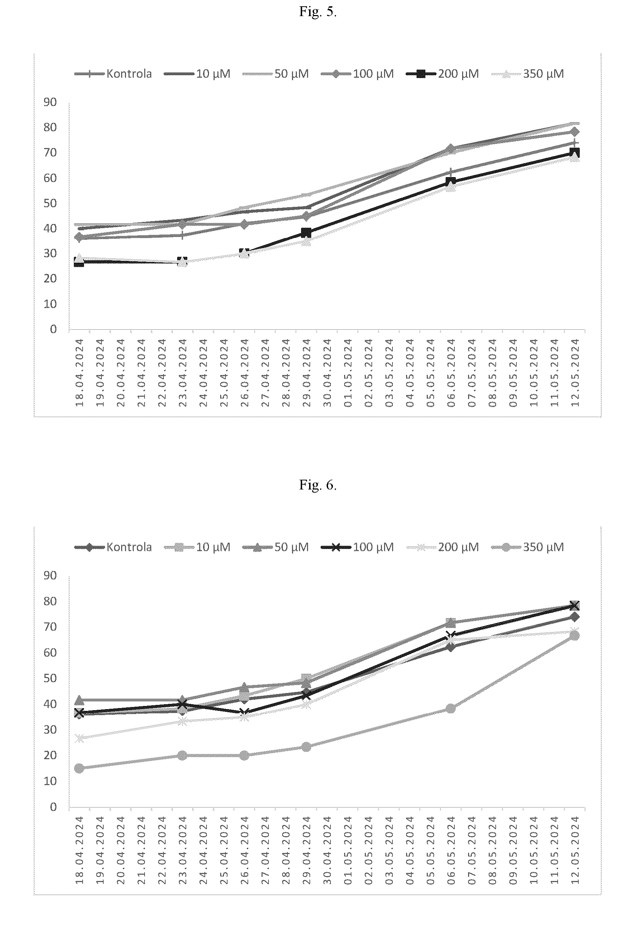

Winter wheat (var. Turandor) was treated in 2024 at a location in Olomouc by spraying substances - designated anti-GIB2-K (potassium (1R,4aR,4bR,7S,8R,9aS,10S,10aR)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate) and anti-GIB2-N (tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The starting substance Anti-GIB2 served as a control. The aim of this test was to find the optimal dosage depending on the developmental stages of winter wheat. The sowing volume was 3.5 million germinated seeds. The experiment was based on 5 randomized replicates for each variant; the control was the untreated variant. The experiment was carried out according to the GEP (Good Experimental Practice) methodology, the size of individual plots was 10 m². The application of the substances was carried out in the amount of 300 l/ha, in the growth phase BBCH 33-34 (middle of stem elongation - 3^{rd} to 4^{th} nodes palpable). The application concentrations were 10, 50, 100, 200 and 350 µM, the amount of spray solution was 300 l/ha. Subsequently, the height of the plants was evaluated at several dates and the length of individual internodes was analyzed. The results are summarized in Tables 11-13 and also in Figures No. 4-6.

**Table 11: Stem length of winter wheat (cm) after application of the control substance Anti-GIB2 (application in BBCH 33-34)**

| APPLICATION DATE | EVALUATION DATE | Control | 10 µM | 50 µM | 100 µM | 200µM | 350 µM |
|---|---|---|---|---|---|---|---|
| 12.04.2024 | 18.04.2024 | 36.00 | 36.67 | 36.67 | 36.67 | 30.00 | 25.00 |
| 12.04.2024 | 23.04.2024 | 37.33 | 43.33 | 36.67 | 36.67 | 33.33 | 26.67 |
| 12.04.2024 | 26.04.2024 | 42.00 | 46.67 | 40.00 | 38.33 | 35.00 | 28.50 |
| 12.04.2024 | 29.04.2024 | 44.67 | 48.33 | 43.33 | 45.00 | 43.33 | 36.67 |
| 12.04.2024 | 06.05.2024 | 62.33 | 70.00 | 70.00 | 66.67 | 65.00 | 55.00 |
| 12.04.2024 | 12.05.2024 | 74.00 | 80.00 | 78.33 | 76.67 | 73.33 | 65.00 |

**Table 12: Stem length of winter wheat (cm) after application of Anti-GIB2-K salt (application in BBCH 33-34)**

| APPLICATION DATE | EVALUATION DATE | Control | 10 µM | 50 µM | 100 µM | 200µM | 350 µM |
|---|---|---|---|---|---|---|---|
| 12.04.2024 | 18.04.2024 | 36.00 | 40.00 | 41.67 | 36.67 | 26.67 | 24.33 |
| 12.04.2024 | 23.04.2024 | 37.33 | 43.33 | 41.67 | 41.67 | 26.67 | 26.67 |
| 12.04.2024 | 26.04.2024 | 42.00 | 46.67 | 48.33 | 41.67 | 30.00 | 30.00 |
| 12.04.2024 | 29.04.2024 | 44.67 | 48.33 | 53.33 | 45.00 | 38.33 | 35.00 |
| 12.04.2024 | 06.05.2024 | 62.33 | 71.67 | 70.00 | 71.67 | 58.33 | 52.67 |
| 12.04.2024 | 12.05.2024 | 74.00 | 81.67 | 81.67 | 78.33 | 70.00 | 58.33 |

**Table 13: Stem length of winter wheat (cm) after application of Anti-GIB2-N salts (application in BBCH 33-34)**

| APPLICATION DATE | EVALUATION DATE | Control | 10 µM | 50 µM | 100 µM | 200 µM | 350 µM |
|---|---|---|---|---|---|---|---|
| 12.04.2024 | 18.04.2024 | 36.00 | 36.67 | 41.67 | 36.67 | 26.67 | 15.00 |
| 12.04.2024 | 23.04.2024 | 37.33 | 38.33 | 41.67 | 40.00 | 33.33 | 20.00 |
| 12.04.2024 | 26.04.2024 | 42.00 | 43.33 | 46.67 | 36.67 | 35.00 | 20.00 |
| 12.04.2024 | 29.04.2024 | 44.67 | 50.00 | 48.33 | 43.33 | 40.00 | 23.33 |
| 12.04.2024 | 06.05.2024 | 62.33 | 71.67 | 71.67 | 66.67 | 65.00 | 38.33 |
| 12.04.2024 | 12.05.2024 | 74.00 | 78.33 | 78.33 | 78.33 | 68.33 | 56.67 |

The results of the application of Anti-GIB2-K and Anti-GIB2-N salts show the dependence of the effect of the derivative on the concentration of the substance in the spray solution. The most effective was the concentration above 100 µM. Logically, the highest concentration of 350 µM was the most effective. The effect of Anti-GIB2-N on shortening the stem length was also significantly manifested. However, the final stem length was most affected by the later application in BBCH 33-34, when the stem shortening effect was noticeable even in the late stages of wheat growth.

### Example 13: Effect of salts of anti-GIB2 applied in BBCH 33-34 phase on wheat growth parameters at harvest

Winter wheat (var. Turandor) was treated in 2024 at a location in Olomouc by spraying substances - designated anti-GIB2-K (potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate) and anti-GIB2-N (tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The starting substance Anti-GIB2 served as a control. The aim of this test was to find the optimal dosage depending on the developmental stages of winter wheat. The sowing volume was 3.5 million germinated seeds. The experiment was based on 5 randomized replicates for each variant; the control was the untreated variant. The experiment was carried out according to the GEP (Good Experimental Practice) methodology, the size of individual plots was 10 m2. The application of substances was carried out in the amount of 300 l/ha, in the growth phase BBCH 33-34 (mid-term of stem elongation - 3^{rd} to 4^{th} palpable nodes). The application concentrations were 100, 200 and 350 µM, the amount of spray solution was 300 l/ha. Subsequently, the growth parameters of wheat plants were evaluated at the time of harvest and the length of individual internodes was analyzed. The results are summarized in Table 14. In Table 15, the measured values are evaluated and expressed in percentages compared to the control.

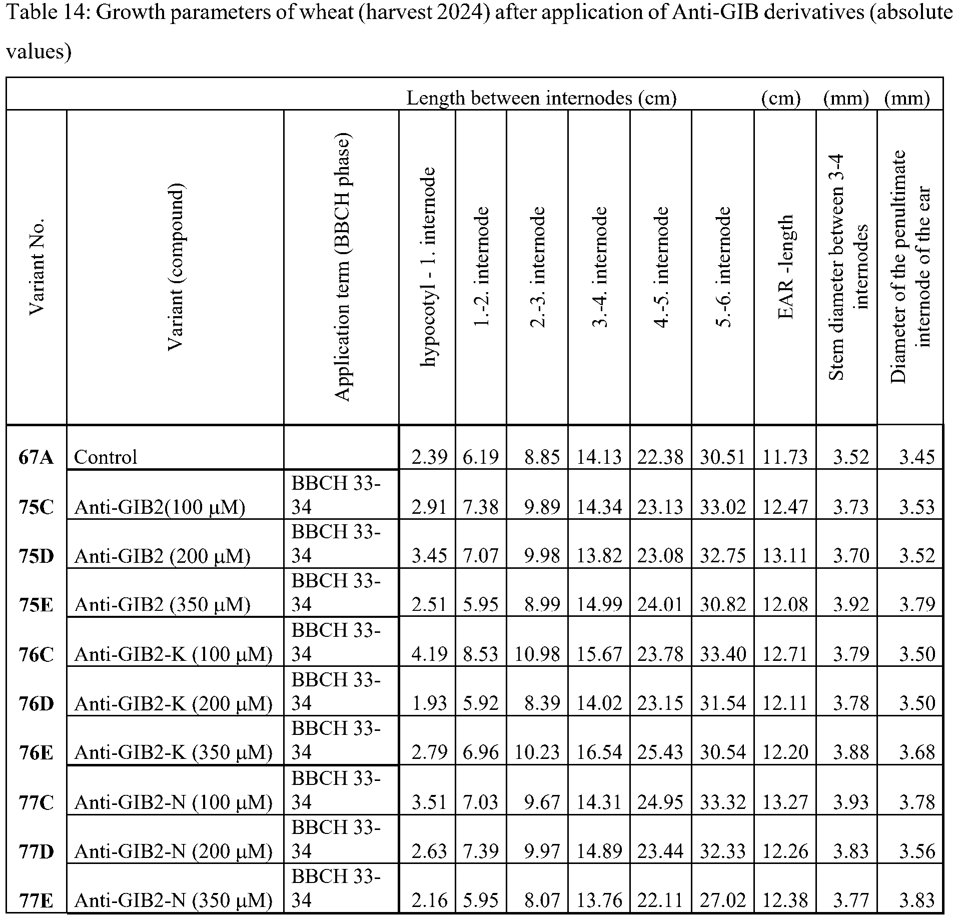

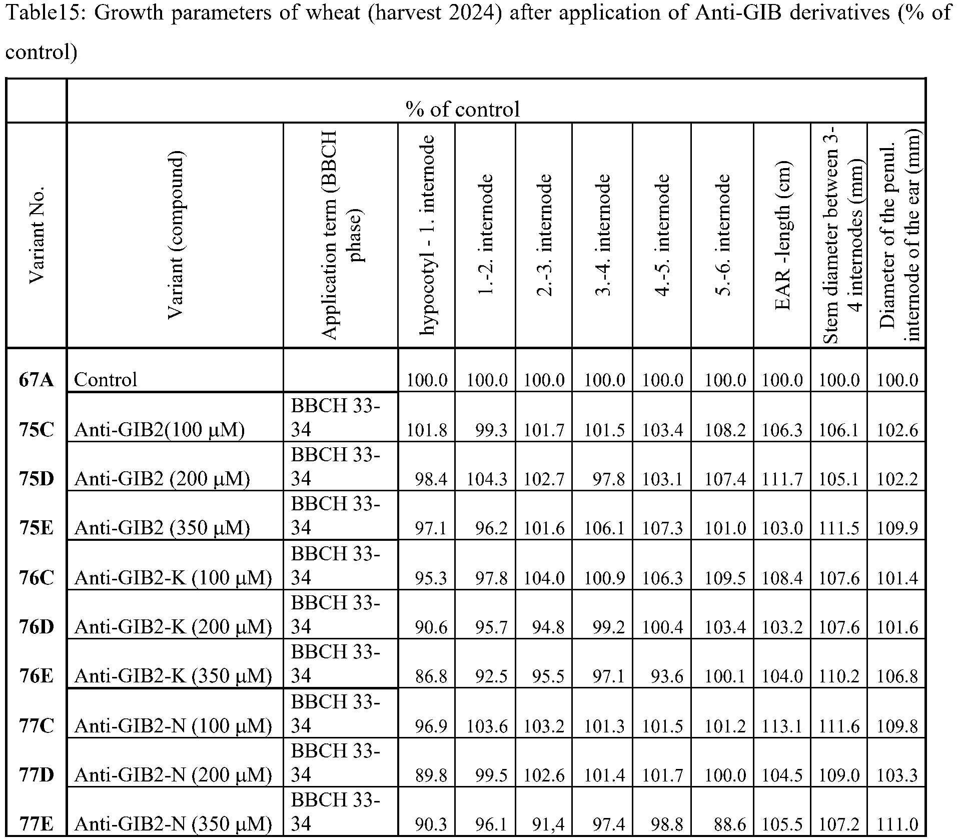

The tested derivatives had an effect on the morphology of the stalk after application - its diameter and the distance between the individual internodes. All applications of Anti-GIB2 substances caused an extension of the ear length by 2.96 to 13.13% over the control, a shortening of the stalk diameter between the 3rd and 4th internodes (by 5.12 - 11.64% over the control) and an increase in the stalk diameter at the penultimate internode of the ear by 1.43 - 11.05% over the control. The tested salts were Anti-GIB2-K and Anti-GIB2-N and were more effective than the starting substance Anti-GIB2. A more pronounced effect was observed especially at higher concentrations, where a higher solubility of the tested salts can be assumed. All these monitored traits have a positive effect on stalk strength, increased lodging resistance and increased grain yield.

### Example 14: Effect of anti-GIB2 salts applied at BBCH 33-34 on yield parameters and total length of wheat during growth and at harvest

Winter wheat (var. Turandor) was treated in 2024 at a location in Olomouc by spraying substances - designated anti-GIB2-K (potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate) and anti-GIB2-N (tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate). The starting substance Anti-GIB2 served as a control. The aim of this test was to find the optimal dosage depending on the developmental stages of winter wheat. The sowing volume was 3.5 million germinating seeds. The experiment was based on 5 randomized replicates for each variant; the control was the untreated variant. The experiment was conducted according to the GEP (Good Experimental Practice) methodology, the size of individual plots was 10 m2. The application of the substances was carried out in the amount of 300 l/ha, in the growth phase BBCH 33-34 (mid-term of stem elongation - 3^{rd} to 4^{th} nodes palpable). The application concentrations were 350 and 500µM, the amount of spray solution was 300 l/ha. Subsequently, the total length of wheat plants was evaluated during the vegetation period and at the time of harvest. The results are summarized in Table No. 16.

**Table 16: Grain yield and height of wheat plants during vegetation and at harvest - harvest 2023**

| | **Variant** | **Grain yield (t/ha)** | **Grain yield (% of control)** | **4.5.** | **9.5.** | **11.5.** | **18.5.** | **22.5.** | **26.5.** | **29.5.** |
|---|---|---|---|---|---|---|---|---|---|---|
| | Control | **13.79** | **100.00** | 45.67 | 57.33 | 61.33 | 73.67 | 80.00 | 89.33 | **95.67** |
| **Anti-GIB2-K** | 350 µM (BBCH 30-31) | **13.87** | **100.58** | 32.67 | 44.00 | 45.00 | 64.00 | 72.67 | 82.67 | **87.00** |
| **Anti-GIB2-K** | 350 µM (BBCH 31-33) | **13.96** | **101.23** | 45.67 | 48.67 | 48.67 | 56.67 | 68.33 | 74.67 | **82.33** |
| **Anti-GIB2-K** | 350 µM (BBCH 34-35) | **14.25** | **103.18** | 46.33 | 59.33 | 60.67 | 61.00 | 67.00 | 69.67 | **75.00** |
| **Anti-GIB2-N** | 350 µM (BBCH 30-31) | **14.01** | **101.59** | 33.00 | 42.33 | 46.67 | 63.60 | 72.67 | 82.33 | **89.00** |
| **Anti-GIB2-N** | 350 µM (BBCH 31-33) | **14.04** | **101.81** | 46.00 | 50.67 | 51.00 | 59.67 | 69.67 | 78.00 | **83.33** |
| **Anti-GIB2-N** | 350 µM (BBCH 34-35) | **14.22** | **103.13** | 50.00 | 61.33 | 62.67 | 66.00 | 68.67 | 73.33 | **77.67** |
| **CCC** | 1,0 1/ha (BBCH 30-31) | **13.40** | **97.22** | 33.67 | 48.68 | 49.00 | 62,33 | 71.00 | 76.00 | **82.00** |
| **Trinexapac-ethyl** | 0,4 l/ha (BBCH 30-31) | **13.16** | **95.46** | 35.33 | 43.00 | 45.00 | 56.33 | 63.00 | 68.67 | **72.76** |

In the 2023 harvest year, Anti-GIB2 and its salts anti-GIB2-K and anti-GIB2-N were applied at concentrations of 350 and 500 µM. All applications shortened the stalk to a similar extent as competitive preparations based on CCC and Trinexapac-ethyl. However, grain yield was clearly above the level of these commercial regulators after the use of salts of the Anti-GIB2 substance. The most significant positive effect on stalk shortening and yield was found at the application date in BBCH 34-35 (yield 103.18 and 103.13% of the control) and the stalk length was 75 and 77.67 cm, compared to 95.67 cm for the control, 82 cm for CCC and 72.76 cm for Trinexapac-ethyl.

## Claims

1. Gibberellin salts of general formula I 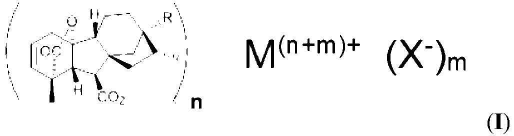 wherein
n+m is 1 or 2;
n is 1 or 2;
m is 0 or 1;
wherein
when n+m is 1, then m is 0 and n is 1, and M⁺ is selected from the group of monovalent alkali metals, monovalent transition metals, and ammonium salts of the general formula N⁺HR'₃, wherein R' are independently selected from C1-C4 alkyls and C1-C4 hydroxyalkyls;
when n+m is 2, then m is 0 or 1 and n is 1 or 2, and M²⁺ is selected from the group of divalent alkaline earth metals and divalent transition metals;
R is -O-C(O)-CH₃ or H; and
X⁻ (if present) is a halide anion or an anion derived from a nitrogen-containing acid.

2. Compound of general formula **I** according to claim 1, selected from the group potassium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate; (tris(2-hydroxyethyl)ammonium (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate; magnesium(II) (1*R*,4a*R*,4b*R*,7*S*,8*R*,9a*S*,10*S*,10a*R*)-7-acetoxy-1,8-dimethyl-13-oxo-1,4,4b,5,6,7,8,9,10,10a-decahydro-4a,1-(epoxymethano)-7,9a-methanobenzo[a]azulene-10-carboxylate.

3. Use of compounds of general formula I according to claim 1 or 2 in agriculture and biotechnology as plant growth regulators.

4. Use of compounds of general formula I according to claim 1 or 2 in agriculture and biotechnology for inhibiting stem elongation of crops, reducing lodging and increasing crop yield.

5. Use according to claim 4, wherein the crops are selected from cereals, oil plants, fibre plants, vegetables, tobacco, nuts, eggplant, sugar cane, tea, grapevine, hops, banana, medicinal plants, and ornamental plants.

6. Use according to claim 4, wherein the crops are selected from the group: wheat, barley, rice, corn, rye, oats, sorghum, rapeseed, mustard, poppy, olives, sunflower, coconut, castor oil, cocoa beans, peanuts, cotton, flax, hemp, jute, spinach, cinnamon, camphor, tobacco, nuts, eggplant, sugar cane, tea, grapevine, hops, banana.

7. Agricultural and/or biotechnological preparations, **characterized in that** they contain at least one compound of general formula I according to claim 1 or 2, and at least one solvent, carrier and/or excipient.
